# FASCICULE DE BREVET EUROPEEN

(11) **EP 0 507 707 B1**
(45) Date de publication et mention de la délivrance du brevet: **12.07.1995**
(21) Numéro de dépôt: 92420104.9
(22) Date de dépôt: 03.04.1992
(51) Int. Cl.: A61M 15/00, A61M 15/08

(54) **Dispositif de commande des fonctions mano-soniques d'un nébuliseur thérapeutique**
Steuervorrichtung eines therapeutischen Verneblers mit Schall-Druck-Funktionen
Driver for generating sound pressure functions in a therapeutic nebulizer

(30) Priorité: 05.04.1991 FR 9104539
(43) Date de publication de la demande: 07.10.1992
(73) Titulaire: LA DIFFUSION TECHNIQUE FRANCAISE S.à.r.l., F-42000 Saint Etienne (Loire) (FR)
(72) Inventeur: Chantrel, Gilles, F-42100 Saint Etienne (FR); Massardier, Michel, F-42000 Saint Etienne (FR)
(74) Mandataire: Perrier, Jean-Pierre

(56) Documents cités:
- DE-A- 3 617 400
- DE-C- 355 416
- US-A- 4 817 626

## Description

L'invention est relative aux nébuliseurs thérapeutiques utilisés pour faire pénétrer des médicaments sous forme d'aérosols dans ce qu'il est convenu d'appeler la sphère O.R.L., et plus particulièrement dans l'oreille moyenne. Elle concerne plus particulièrement les nébuliseurs comprenant un embout narinaire se logeant de manière étanche dans les narines du sujet à traiter et mettant à profit la déglutition du sujet pour introduire, à travers les trompes d'Eustache, ouvertes par cette déglutition, un aérosol thérapeutique, au moins temporairement mis simultanément en vibration et en surpression.

De tels dispositifs sont décrits dans le brevet français 1 567 403 et dans la demande de brevet français 2 639 236 au nom de la demanderesse. La fonction sonique, c'est à dire la mise en vibrations de la membrane pour générer des vibrations soniques, et la fonction de mise en surpression temporaire, sont déclenchées simultanément par un dispositif de commande interposé entre le nébuliseur, le circuit d'aspiration d'une pompe à membrane, un circuit d'alimentation en air dérivé de celui allant de la pompe à membrane au nébuliseur et comportant un réservoir d'accumulation.

Le déclenchement du dispositif de commande est assuré par le sujet en cours de traitement qui doit intervenir sur un moyen approprié, en même temps qu'il déglutit pour former dans la sphère O.R.L. une enceinte étanche permettant d'obtenir, dans cette enceinte et jusqu'à une chambre appropriée du dispositif de commande, une surpression de pilotage. Cette surpression pilote le dispositif de commande dans le sens du déclenchement simultané des fonctions manosoniques.

Plus précisément, la présente invention est en rapport avec la forme d'exécution du dispositif de commande décrit dans cette demande de brevet, comprenant, d'une part, une chambre d'émission de vibrations soniques, raccordée par un circuit de traitement au conduit de sortie du nébuliseur et séparée, par une membrane, d'une chambre de génération de vibrations, d'autre part, une chambre d'aspiration qui, raccordée à un générateur d'air pulsé, communique avec la chambre de génération des vibrations par une ouverture obturée par un clapet et est séparée, par une membrane déformable liée à la tige du clapet précité, d'une chambre de commande raccordée au circuit d'alimentation principal, de plus, un réservoir d'accumulation alimenté par un circuit dérivé du circuit d'alimentation principal, et apte à débiter dans le circuit de traitement, et enfin des moyens de déclenchement d'une pression de pilotage de la surpression.

Il s'avère à l'usage que le traitement thérapeutique serait plus efficace si la phase de mise en vibrations soniques de l'aérosol pouvait être séparée de sa phase de mise en surpression et pouvait être intercalée entre la phase de génération de l'aérosol et celle de traitement complet par aérosols mis en vibrations soniques et en surpression.

Cette modification du traitement ne peut pas être mis en oeuvre avec les dispositifs actuels, ne tolérant que l'émission d'aérosols simples ou un traitement complet.

L'objet de l'invention est de fournir un dispositif de commande qui permette, non seulement, d'obtenir la simultanéité des fonctions soniques de mise en surpression, dans la phase de traitement complet, mais aussi d'intercaler entre la génération d'aérosol et le traitement complet, une phase complémentaire de mise en vibrations soniques de l'aérosol et cela avec des moyens simples, peu encombrants, fiables et dont la mise en oeuvre soit aisée et sans contraintes de synchronisation de la commande pour le sujet à traiter.

A cet effet, dans le dispositif de commande selon l'invention, la chambre de commande de la génération des vibrations soniques est distincte de la chambre de commande de la mise en surpression et est raccordée, d'un côté, à la source d'air pulsé et, d'un autre côté, à un circuit d'échappement comportant des moyens de fermeture réagissant à la présence d'une surpression dans un circuit dérivé du circuit d'alimentation en air pulsé du nébuliseur, tandis que, seule, la chambre de commande de la mise en surpression est raccordée par un circuit, de pilotage et de transfert, au circuit de traitement, cette dernière chambre étant séparée d'une chambre d'équilibrage par une membrane assurant l'obturation d'une buse raccordée au réservoir.

Avec cet agencement, la génération des vibrations par l'air pulsé, est déclenchée après génération de l'aérosol et seulement alors que la pression dans le circuit dérivé est suffisante, et cela de manière automatique, sans intervention du sujet traité, alors que le traitement complet, incluant génération d'aérosols, la mise en vibrations soniques de ceux-ci et la mise en surpression du circuit de traitement, nécessite un déclenchement par le patient. Ce déclenchement, effectué par déglutition du patient et actionnement des moyens de déclenchement, et réalisé par le circuit pilotant la chambre de commande de la mise en surpression, provoque, par déplacement de la membrane, l'ouverture de la buse raccordée au réservoir. Cela provoque l'injection de l'air en surpression du réservoir dans le circuit de traitement, par l'intermédiaire du circuit de pilotage devenant circuit de transfert de la surpression. Dans ces conditions, la surpression s'ajoute à la fonction sonique pour permettre à l'aérosol de franchir la trompe d'Eustache et traiter efficacement l'oreille moyenne. La vidange du réservoir conduit à une baisse progressive de la pression dans le réservoir et dans son circuit d'alimentation, ce qui provoque ainsi, par les moyens de fermeture du circuit d'échappement, la mise à l'air libre de la chambre de commande des vibrations. Il en résulte, qu'après un certain temps, le traitement complet s'interrompt par disparition de la surpression et des vibrations, ce qui incite le patient à recommencer le traitement, en suivant la succession des phases imposées par le dispositif de commande.

D'autres caractéristiques et avantages ressortiront de la description qui suit en référence au dessin schématique annexé représentant, à titre d'exemple non limitatif, une forme d'exécution de ce dispositif de commande.

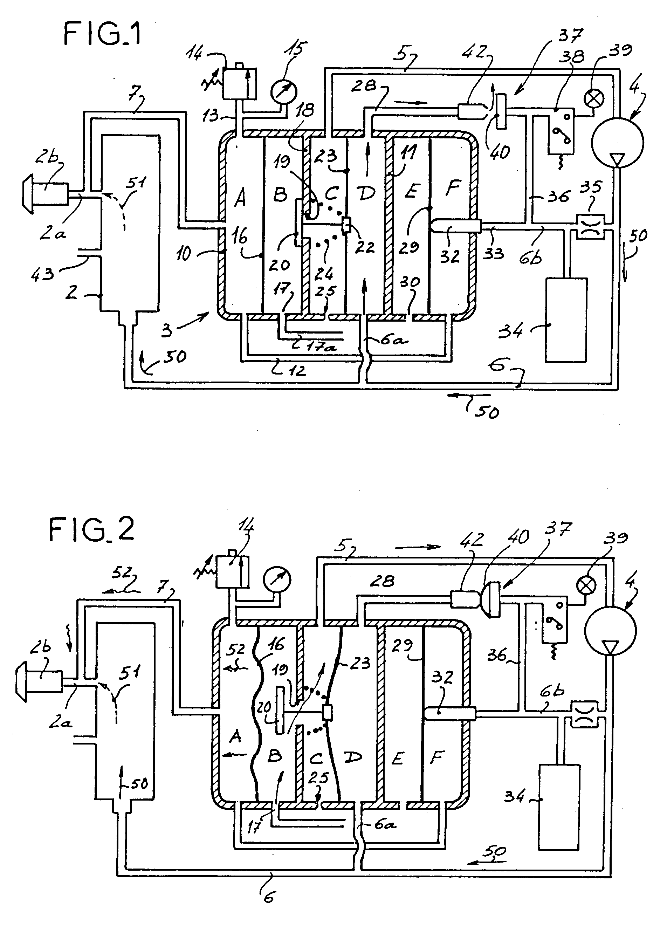

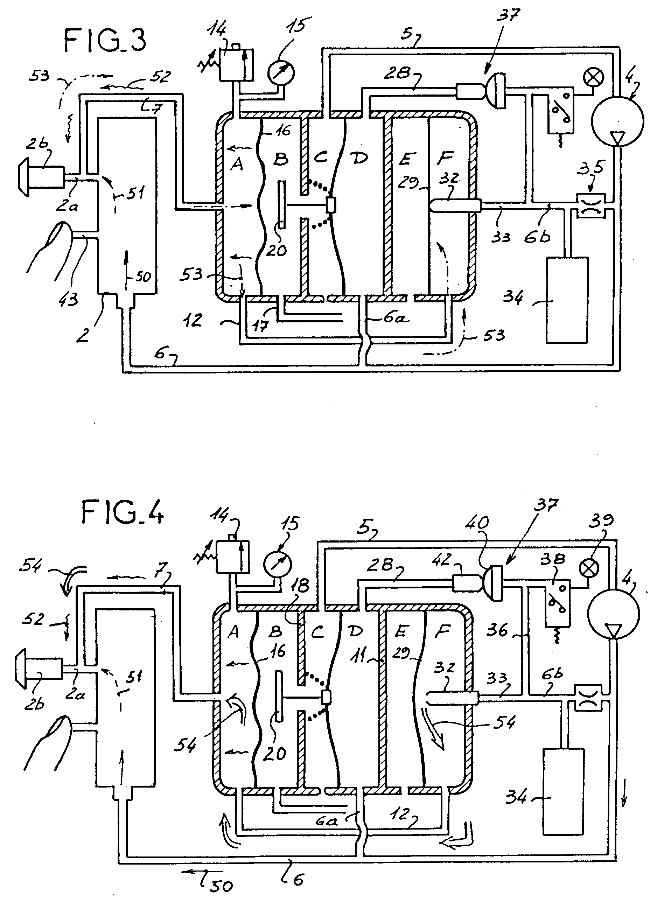

Figures 1 à 4 sont des vues schématiques de l'ensemble de l'installation lorsque son dispositif de commande est, respectivement :
- pour la figure 1, dans la phase de mise en pression au démarrage,
- pour la figure 2, dans la phase d'émission des vibrations,
- pour la figure 3, dans la phase de pilotage du traitement complet,
- et pour la figure 4, pendant la phase du traitement complet.

Cette installation de traitement est donc composée d'un nébuliseur désigné par la référence 2, d'un boîtier de commande 3, d'un générateur 4 d'air pulsé, et en l'occurrence une pompe électromagnétique à membrane.

La pompe 4 est alimentée par un circuit d'aspiration 5 et débite dans un circuit principal 6 constituant circuit d'alimentation du nébuliseur 2.

Le nébuliseur est du type muni d'un conduit de sortie 2a raccordable à un embout narinaire 2b, étanche, amovible et raccordé en permanence à un circuit de traitement 7 provenant du dispositif de commande 3.

Le dispositif de commande 3 est composé d'un boîtier 10 dans lequel des cloisons 11 et 18, et des membranes élastiques déformables 16-23-29 délimitent plusieurs chambres, à savoir une chambre A d'émission des vibrations, une chambre B de génération des vibrations, une chambre C d'aspiration, une chambre D de commande des vibrations, une chambre E d'équilibrage et une chambre F de commande de la mise en surpression.

La chambre A d'émission des vibrations est raccordée au circuit de traitement 7 et, par un conduit 12, dit de pilotage et de transfert, à la chambre F de commande de la mise en surpression. Elle communique aussi par un conduit 13, d'une part, avec une soupape de décharge 14 avec réglage de son tarage, et, d'autre part, avec un manomètre 15 d'indication de la pression dans le circuit de traitement. Elle est séparée de la chambre B de génération des vibrations par la membrane déformable 16.

La chambre B de génération des vibrations communique avec l'extérieur par un ajutage 17 pouvant être associé à un conduit d'amplification 17a. Elle est séparée de la chambre C d'aspiration par la paroi rigide 18 comportant une ouverture centrale 19 obturée par un clapet 20. La tige de ce clapet traverse l'ouverture 19 et la totalité de la chambre C d'aspiration, et est fixée, en 22, sur la membrane 23 séparant la chambre C d'aspiration de la chambre D de commande de mise en vibrations. Un ressort de compression 24, interposé entre la paroi 18 et la membrane 23, assure le placage du clapet 20 contre la paroi 18 pour obturer l'ouverture 19.

La chambre C d'aspiration est raccordée, d'un côté, au circuit d'aspiration 5 allant à la pompe 4, et, d'un autre côté, à l'air libre par un ajutage calibré 25 ayant, de façon connue, un diamètre plus petit que celui de l'ajutage 17 de la chambre D.

La chambre D de commande de la mise en vibrations qui, selon une caractéristique de l'invention, n'assure que cette fonction, est raccordée, d'un côté, à une branche 6a du circuit d'alimentation 6 et, d'un autre côté, à un circuit d'échappement 28, muni de moyens de fermeture dont il sera fait état plus loin.

La chambre F de commande de la mise en surpression du circuit de traitement, chambre qui n'assure, suivant une autre caractéristique de l'invention, que cette seule fonction, est séparée, par la membrane déformable 29, d'une chambre d'équilibrage E, communiquant avec l'extérieur par un orifice 30.

La membrane 29 constitue moyen de fermeture d'une buse 32, raccordée par un conduit 33, à un réservoir d'accumulation 34. Ce réservoir est alimenté en air pulsé, et à travers un ajutage calibré 35, par un circuit 6b dérivé du circuit principal 6.

Le circuit dérivé 6b est raccordé au conduit 33, qui en constitue l'une des branches, et à un conduit 36 qui en constitue une autre branche. Ce conduit 36 alimente des moyens de fermeture pneumatique 37 du circuit d'échappement 28 précité, et un contacteur pneumatique 38 d'actionnement d'un voyant lumineux 39.

Le moyen de fermeture 37 est composé, dans la forme d'exécution représentée, par un boîtier dont l'une des parois est constituée par une membrane élastique déformable 40 qui, disposée en vis à vis d'une buse 42 ménagée à l'extrémité du circuit d'échappement 28, est apte à obturer cette buse.

Enfin, de façon connue, ce dispositif de commande est muni de moyens de déclenchement qui, dans la forme d'exécution représentée, sont constitués par un ajutage 43 ménagé dans le corps du nébuliseur 2 et formant une prise d'air additionnelle.

Lorsque l'installation de traitement est à l'arrêt, comme montré à la figure 1, toutes les membranes 16-23-29 sont en position de repos, le clapet 20 obture l'ouverture 19, la membrane 29 obture la buse 32, tandis que la membrane 40 dégage la buse 42.

A la mise en route de l'installation, la pompe 4 débite dans le circuit d'alimentation 6 un courant d'air pulsé qui, comme montré par les flèches 50 à la figure 1, va au corps du nébuliseur 2, préalablement rempli par le liquide de traitement, et assure, par pulvérisation, la formation d'un aérosol thérapeutique comme montré par la flèche 51, figure 1. Par le conduit 2a, cet aérosol va à l'embout narinaire préalablement introduit, de manière étanche, dans les narines du sujet. Simultanément, mais progressivement en raison de l'ajutage calibré 35, le circuit dérivé 6b-33-36 est alimenté en air pulsé. En l'absence de fuite, ce circuit est soumis progressivement à une augmentation de sa pression. Cette augmentation de pression favorise l'accumulation de l'air dans le réservoir 34 et provoque, progressivement, la déformation de la membrane 40 des moyens de fermeture 37, jusqu'à ce que cette membrane vienne obturer la buse 42 et fermer ainsi le circuit d'échappement 28 de la chambre D de commande des vibrations.

A partir de cette fermeture, et comme montré figure 2, l'air pulsé pénétrant dans la chambre de commande D, par la branche 6a du circuit d'alimentation 6, est soumis à une augmentation de sa pression, augmentation qui entraîne la déformation de la membrane 23, avec, pour conséquence, le déplacement du clapet 20 et le dégagement de l'ouverture 19 ménagée dans la cloison 18. L'alimentation du circuit d'aspiration 5, qui s'effectuait jusqu'alors par l'orifice calibré 25 de la chambre d'aspiration C, s'effectue alors à travers l'orifice 17 de plus grand diamètre de la chambre B et, de là, par l'ouverture 19 de la cloison 18.

De ce fait, et en raison du caractère pulsé de l'aspiration procurée par la pompe 4, la membrane 16, séparant les chambres A et B, est animée d'un mouvement vibratoire à une fréquence double de la fréquence des pulsations de la pompe 4, mouvement qui se transmet, par la chambre A et le circuit de traitement 7, jusqu'aux gouttelettes d'aérosol traversant le conduit de sortie 2a du nébuliseur, comme montré par les flèches 52.

Il faut noter ici que la commande de génération des vibrations est effectuée, de manière entièrement automatique, après la mise en route de l'installation et la génération de l'aérosol, mais avec une temporisation, qui dépend du temps nécessaire pour actionner les moyens de fermeture 37, c'est à dire pour déformer la membrane 40, donc du temps que met le circuit dérivé pour monter à une pression suffisante.

Dans cet état, l'installation de traitement peut fonctionner sans interruption, en délivrant un produit thérapeutique sous forme d'aérosols mis en vibrations. Il ne s'agit là que d'une faculté, car cette fonction ne présente qu'un intérêt temporaire par le seul fait que l'efficacité du traitement est accrue par une mise en pression, simultanée et ponctuelle.

C'est pour informer que l'installation est prête à effectuer cette action simultanée et ponctuelle, que le contacteur pneumatique 38 déclenche l'alimentation électrique du voyant lumineux 39, lorsque est suffisante la pression dans le circuit dérivé 6b-33-36 mais aussi dans le réservoir 34.

A ce stade, et comme montré à la figure 3, le déclenchement du traitement complet est effectué par le patient qui doit, simultanément ou successivement, obturer avec un doigt l'ajutage 43, ménagé sur le corps 2 du nébuliseur, et déglutir, afin de mettre en oeuvre le mécanisme physiologique par lequel le voile du palais vient s'appliquer, au niveau des choanes, sur le débouché des fosses nasales pour obturer celui-ci, en séparant ainsi temporairement ces fosses du circuit bucco-pharyngien. L'insufflation du débit d'air continue dans les fosses nasales, à l'aide de l'embout 2b ajusté de façon étanche dans les narines, produit, au moment de cette déglutition et en raison de la fermeture étanche du circuit bucco-pharyngien, une surpression dans le circuit d'inhalation. Cette surpression, transmise dans le circuit de traitement 7 à la chambre A constitue la pression de pilotage qui, à travers le circuit de pilotage 12, et comme montré par les flèches 53, est amenée dans la chambre F de commande de la mise en surpression du circuit de traitement pour provoquer la déformation de la membrane 29 et, en conséquence, l'ouverture de la buse 32.

Il faut noter que la valeur de la pression de pilotage est très faible, et par exemple inférieure à 10 millibars, puisqu'elle correspond à la seule résistance à la déformation de la membrane 29.

Dès que la membrane 29 libère la buse 32, et comme montré figure 4, l'air en surpression dans le circuit dérivé, mais aussi dans le réservoir 34, par exemple à une pression de 350 millibars, pénètre dans la chambre F, puis, par le conduit 12, formant alors conduit de transfert et comme montré par les flèches 54, dans la chambre A et, de là, par le conduit de traitement 7, le conduit de sortie 2a et l'embout narinaire 2b, dans la cavité bucco-pharyngée. La déglutition étant maintenue, l'aérosol, mis en vibrations soniques et en surpression, force le passage à travers les trompes d'Eustache en position ouverte et vient ainsi traiter, avec efficacité, l'oreille moyenne.

La valeur maximale de la surpression empruntant le circuit de traitement 7 est limitée par la soupape de décharge 14 à une valeur réglable comprise entre 0 et 50 millibars,mesurée par le manomètre 15.

Au fur et à mesure que l'air provenant du réservoir est débité dans le circuit de traitement 7, la pression dans le circuit de dérivation 6b-33-36 baisse, et provoque par le manocontact 38, l'interruption de l'allumage du voyant lumineux 39 et par la membrane 40 des moyens 37, l'ouverture de la buse 42 du circuit d'échappement de la chambre D.

Il en résulte que l'émission de vibrations cesse, que la surpression disparaît et que le sujet peut arrêter de déglutir et libérer la prise d'air additionnelle 43 du nébuliseur 2.

Un nouveau cycle peut reprendre avec, successivement, génération de l'aérosol thérapeutique, mise en vibrations de cet aérosol, et adjonction de la surpression.

Il ressort de ce qui précède que le dispositif de commande, selon l'invention, est simple à réaliser, a un fonctionnement simple et sûr, parfaitement adapté aux besoins, et ne met en oeuvre qu'une seule pompe vibrante pour produire l'air comprimé et pulsé qui crée l'aérosol, génère les vibrations soniques, et alimente la réserve d'air provoquant la surpression. En outre, la mise en oeuvre de ce dispositif est entièrement automatique pour les deux premières phases du traitement et ne nécessite, pour la dernière phase de traitement complet, qu'une manoeuvre volontaire, d'autant plus simple à réaliser, même par un enfant, que c'est la déglutition qui déclenche la pression de pilotage et qu'il n'est donc pas nécessaire de synchroniser parfaitement la déglutition avec la fermeture de la prise d'air additionnelle.

Il est évident que le dispositif de fermeture 37 du circuit d'échappement 28 de la chambre de commande des vibrations, dispositif qui a été décrit ci-dessus comme étant pneumatique, peut, dans une variante de réalisation, être remplacé par une vanne électrique à commande par manocontact.

## Revendications

1. Dispositif de commande des fonctions mano-soniques d'un nébuliseur thérapeutique, du type comprenant, d'une part, une chambre (A) d'émission de vibrations soniques, raccordée par un circuit de traitement (7) à l'embout narinaire étanche (2b) d'un nébuliseur (2) et séparée, par une membrane (16), d'une chambre (B) de génération des vibrations, d'autre part, une chambre (C) d'aspiration qui, raccordée par un circuit (5) à un générateur (4) d'air pulsé, communique avec la chambre (B) de génération des vibrations, par une ouverture (19) obturée par un clapet (20), et est séparée d'une chambre (D) de commande par une membrane déformable (23), liée à la tige du clapet (20) précité, cette chambre (D) étant alimentée par le générateur (4) au moyen d'un circuit (6-6a), de plus, un réservoir d'accumulation (34) alimenté par un circuit (6b), dérivé du circuit d'alimentation principal (6), et apte à débiter dans le circuit de traitement (7), et, enfin, des moyens (43) de déclenchement d'une pression de pilotage de la surpression, **caractérisé en ce que** la chambre (D) de commande de la génération des vibrations soniques est distincte de la chambre (F) de commande de la mise en surpression et est raccordée, d'un côté, à la source (4) d'air pulsé et, d'un autre côté, à un circuit d'échappement (28) comportant des moyens de fermeture (37) réagissant à la présence d'une surpression dans un circuit dérivé (6b) du circuit d'alimentation (6) en air pulsé du nébuliseur (2), tandis que, seule, la chambre (F) de commande de la mise en surpression est raccordée par un circuit (12), de pilotage et de transfert, au circuit de traitement (7), cette dernière chambre (F) étant séparée d'une chambre d'équilibrage (E) par une membrane (29) assurant l'obturation d'une buse (32) raccordée au réservoir (34).

2. Dispositif selon la revendication 1, **caractérisé en ce que** le moyen de fermeture (37) du circuit d'échappement (28) de la chambre D de commande de la mise en vibrations, est constitué par un boîtier raccordé à un circuit d'alimentation (36) et présentant au moins une paroi constituée par une membrane (40), cette membrane étant disposée en vis à vis d'une buse (42) fixée à l'extrémité du circuit d'échappement (28) et de manière à pouvoir obturer cette buse de façon étanche.

3. Dispositif selon l'une quelconque des revendications 1 et 2, **caractérisé en ce que** le circuit (33) d'alimentation de la buse (32) de la chambre (F) de commande de la mise en surpression, celui d'alimentation (36) des moyens de fermeture (37) du circuit d'échappement (28), de la chambre (D) de commande de la mise en vibrations, et celui du réservoir (34) sont alimentés par un même circuit (6b) monté, à travers un ajutage calibré (35), en dérivation du circuit d'alimentation (6) du nébuliseur (2) et de la chambre (D) de commande de la mise en vibrations.

4. Dispositif de commande selon l'une quelconque des revendications 1 à 3, **caractérisé en ce que** le circuit (6b-36) dérivé du circuit d'alimentation (6) est muni d'un contacteur pneumatique (38) alimentant un voyant lumineux (39) d'indication de la disponibilité de la surpression dans le réservoir (34).

## Claims

1. A device for controlling the mano-sonic functions of a therapeutic nebuliser, of the type having, on the one hand, a chamber (A) for emitting sonic vibrations, connected by a treatment circuit (7) to the sealed inhaler nozzle (2b) of a nebuliser (2) and separated, by a membrane (16), from a chamber (B) for generating vibrations, on the other hand, a suction chamber (C) which, being connected by a circuit (5) to a pulsed air generator (4), communicates with the vibration generating chamber (B) by an opening (19) closed by a valve (20) and is separated from a control chamber (D) by a deformable membrane (23), connected to the rod of the aforesaid valve (20), this chamber (D) being supplied by the generator (4) by means of a circuit (6-6a), in addition an accumulator reservoir (34) supplied by a circuit (6b) branched from the main supply circuit (6), and adapted to discharge into the treatment circuit (7) and, finally, means (43) for triggering a control pressure for the boost pressure, characterised in that the chamber (D) for controlling the generation of sonic vibrations is distinct from the chamber (F) controlling the establishment of boost pressure and is connected, on the one hand, to the source (4) of pulsed air and on the other hand, to an exhaust circuit (28) having closure means (37) reacting to the presence of a pressure increase in a circuit (6b) branched from the circuit (6) supplying pulsed air to the nebuliser (2), whilst only the chamber (F) controlling the establishment of boost pressure is connected by a control and transfer circuit (12) to the treatment circuit (7), this latter chamber (F) being separated from a balance chamber (E) by a membrane (29) ensuring the closure of a nozzle (32) connected to the reservoir (34).

2. A device according to Claim 1, characterised in that the closure means (37) of the exhaust circuit (28) of the chamber (D) controlling the establishment of vibrations, is constituted by a housing connected to a supply circuit (36) and having at least one wall constituted by a membrane (40), this membrane being disposed opposite to a nozzle (42) fixed to the end of the exhaust circuit (28) so as to be able to close this nozzle in sealing manner.

3. A device according to either of Claims 1 and 2, characterised in that the supply circuit (33) for the nozzle (32) of the chamber (F) controlling the establishment of boost pressure, that (36) for supplying the closure means (37) of the exhaust circuit (28) of the chamber (D) controlling the establishment of vibrations, and that of the reservoir (34) are supplied by the same circuit (6b) mounted across a calibrated restriction (35) branched from the supply circuit (6) of the nebuliser (2) and of the chamber (D) controlling the establishment of vibrations.

4. A control device according to any one of Claims 1 to 3, characterised in that the circuit (6b-36) branched from the supply circuit (6) is provided with a pneumatic switch (38) supplying a light indicator (39) indicating the availability of boost pressure in the reservoir (34).

## Patentansprüche

1. Vorrichtung zum Steuern der Druck-Schall-Funktionen eines therapeutischen Zerstäubers, umfassend einerseits eine Kammer (A) zur Emission von Schallschwingungen, die mittels einer Behandlungsleitung (7) an das dichte Nasen-Ansetzstück (2b) eines Zerstäubers (2) angeschlossen ist und durch eine Membrane (16) von einer Kammer (B) zur Erzeugung der Schwingungen getrennt ist, andererseits eine Ansaugkammer (C), die über eine Leitung (5) an einen Generator (4) für gepulste Luft angeschlossen ist, mit der Kammer (B) zur Erzeugung der Schwingungen über eine von einem Ventilkörper (20) verschlossene Öffnung (19) in Verbindung steht, und von einer Steuerkammer (D) durch eine verformbare Membrane (23) getrennt ist, wobei die verformbare Membrane (23) mit dem Schaft des Vorgenannten Ventilkörpers (20) verbunden ist, wobei diese Kammer (D) von dem Generator (4) über eine Leitung (6-6a) gespeist ist, ferner ein Sammelreservoir (34), das über eine von der Hauptspeiseleitung (6) abgezweigte Leitung (6b) gespeist ist und in die Behandlungsleitung (7) abgeben kann, und schließlich Mittel (43) zum Auslösen eines Drucks zum Steuern des Überdrucks, **dadurch gekennzeichnet**, daß die Kammer (D) zum Steuern der Erzeugung der Schallschwingungen von der Kammer (F) zum Steuern des Unter-Überdruck-Setzens verschieden ist und einerseits an die Quelle (4) für gepulste Luft und andererseits an eine Auslaßleitung (28) angeschlossen ist, wobei die Auslaßleitung (28) Schließmittel (37) umfaßt, die auf das Vorliegen eines Überdrucks in einer von der Leitung (6) zum Speisen des Zerstäubers (2) mit gepulster Luft abgezweigten Leitung (6b) reagieren, während nur die Kammer (F) zum Steuern des Unter-Überdruck-Setzens über eine Steuer- und Transfer-Leitung (12) an die Behandlungsleitung (7) angeschlossen ist, wobei diese letzere Kammer (F) von einer Ausgleichskammer (E) mittels einer Membrane (29) getrennt ist, welche das Verschließen einer an das Reservoir (34) angeschlossenen Düse (32) sicherstellt.

2. Vorrichtung nach Anspruch 1, **dadurch gekennzeichnet**, daß das Schließmittel (37) zum Verschließen der Auslaßleitung (28) der Kammer (D) zum Steuern des In-Schwingungen-Versetzens von einem Gehäuse gebildet ist, das an eine Speiseleitung (36) angeschlossen ist und wenigstens eine Wandung aufweist, die von einer Membrane (40) gebildet ist, wobei diese Membrane einer am Ende der Auslaßleitung (28) befestigten Düse (42) gegenüberliegend angeordnet ist derart, daß sie diese Düse dichtend abdecken kann.

3. Vorrichtung nach einem der Ansprüche 1 und 2, **dadurch gekennzeichnet**, daß die Leitung (33) zum Speisen der Düse (32) der Kammer (F) zum Steuern des Unter-Überdruck-Setzens, jene (36) zum Speisen der Schließmittel (37) zum Verschließen der Auslaßleitung (28) der Kammer (D) zum Steuern des In-Schwingungen-Versetzens und jene des Reservoirs (34) von ein und derselben Leitung (6b) gespeist sind, die über eine kalibrierte Öffnung (35) von der Speiseleitung (6) des Zerstäubers (2) und der Kammer (D) zum Steuern des In-Schwingungen-Versetzens abgezweigt ist.

4. Vorrichtung nach einem der Ansprüche 1 bin 3, **dadurch gekennzeichnet**, daß die von der Speiseleitung (6) abgezweigte Leitung (6b-36) mit einem Pneumatikkontakt (38) versehen ist, der eine Leuchtanzeige (39) zur Anzeige der Verfügharkeit des Überdrucks in dem Reservoir (34) speist.
